# EUROPEAN PATENT SPECIFICATION

(11) **EP 2 002 734 B1**
(45) Date of publication and mention of the grant of the patent: **05.10.2016**
(21) Application number: 07011615.7
(22) Date of filing: 13.06.2007
(51) Int. Cl.: A23L 29/30, A23L 27/30, A23L 2/02, A23L 2/60, A23C 9/13, A61P 3/10, A61P 3/06, A61P 9/02, C13B 50/00, C13B 10/00, A61K 31/70

(54) **CARBOHYDRATE COMPOSITION OBTAINABLE FROM MEDITERRANEAN FRUITS**
AUS MEDITERRANEN FRÜCHTEN GEWINNBARE KOHLEHYDRATZUSAMMENSETZUNG
COMPOSITION DE GLUCIDE OBTENUE À PARTIR DE FRUITS MÉDITERRANÉENS

(43) Date of publication of application: 17.12.2008
(73) Proprietor: Wild Valencia, S.A., 46740 Carcaixent Valencia (ES)
(72) Inventor: Wild, Hans-Peter, 69214 Eppelheim (DE); Salom, Rafael, 46250 L'Alcudia (ES); Zaldua, Ignacio, 46003 Valencia (ES)
(74) Representative: Grünecker Patent- und Rechtsanwälte PartG mbB

(56) References cited:
- EP-A- 0 440 586
- EP-A- 0 587 972
- EP-A- 0 617 133
- EP-A- 0 765 609
- WO-A-00/60128
- WO-A-88/01835
- WO-A-02/078458
- WO-A-2007/062087
- DE-A1- 19 619 844
- FR-A- 2 157 819
- GB-A- 716 934
- US-A- 4 296 141

## Description

The present invention is directed to a composition comprising carbohydrates obtainable from carob and carbohydrates obtainable from at least one further fruit.

The use of natural fruit-derived products has become more important in recent years in view of increasing health consciousness.

In order to contribute to the human health it is desirable that foodstuff has a low glycaemic index (GI) within a balanced diet and a healthy lifestyle. The GI was first introduced in 1981 and is a classification of the blood glucose raising potential of carbohydrate foods. It is defined as the incremental area under the blood glucose curve of a 50 g carbohydrate portion of a test food expressed as a percentage of the response to 50 g of a reference food taken by the same subject on a different day.

Carbohydrate foods consumed in isoglucidic amounts produce different glycaemic responses depending on many factors, such as particle size, cooking and food processing, other food components (e.g., fat, protein, dietary fibre) and starch structure. The principle is that the slower the rate of carbohydrate absorption, the lower the rise of blood glucose level and the lower the GI value. High-GI foods are characterised by fast-release carbohydrate and higher blood glucose levels. A GI value ≥ 70 is considered high, a GI value 56-69 inclusive is medium and a GI value ≤ 55 is low (where glucose = 100).

The GI of foods has important implications for the prevention and treatment of the major causes of morbidity and mortality in Western countries, including Type 2 Diabetes, cardiovascular disease, coronary heart disease, obesity, and hyperlipidemia. In addition, low GI foods have been associated with prolonged endurance during physical activity, improved insulin sensitivity and increase in colonic fermentation.

WO 02/078458 describes a composition comprising a mixture of sugars (sucrose, fructose, lactose) with low glycemic index. Used in food and pharmaceuticals to prevent obesity and diabetes. EP-A-0 587 972 describes a composition/beverage comprising a mixture of fruit juices (sucrose, fructose, glucose and other carbohydrates) as source of natural sweetener. EP-A-0617133 describes a natural sweetener comprising sugars (sucrose, fructose, glucose and other carbohydrates) from carob fruit. The fruit juice is pressed to pulp, separated (filtering, centrifuge and refined by exchange resins).

The problem underlying the present invention is to provide a composition allowing the preparation of foodstuff having a low GI and a pleasant taste.

Said problem is solved by a composition, comprising carbohydrates obtainable from carob and carbohydrates obtainable from at least one further fruit, comprising 18-28% (w/w) of glucose, 30-44% (w/w) of fructose, 16-33% (w/w) of saccharose, 7-13% (w/ w) of polyalcohols, and 1-3% (w/w) of other sugars, wherein the percentages are based on the dry matter.

In a preferred embodiment the further fruit is a Mediterranean fruit.

In another preferred embodiment the composition comprises carbohydrates obtainable from carob and apple, more preferably 40-60% (w/w) carbohydrates obtainable from carob, and 40-60% (w/w) carbohydrates obtainable from apple, wherein the percentages are based on the dry matter.

The composition comprises in a further preferred embodiment carbohydrates obtainable from carob, apple and grape, more preferably 35-45% (w/w) carbohydrates obtainable from carob, 40-50% (w/w) carbohydrates obtainable from apple, and 10-20% (w/w) carbohydrates obtainable from grape, in particular 40% (w/w) carbohydrates obtainable from carob, 45% (w/w) carbohydrates obtainable from apple, and 15% (w/w) carbohydrates obtainable from grape, wherein the percentages are based on the dry matter. The grape is preferably white grape.

Preferably, the composition comprises carbohydrates obtainable from carob, apple, grape and pear, in particular 35-45% (w/w) carbohydrates obtainable from carob, 35-45% (w/w) carbohydrates obtainable from apple, 5-15 % (w/w) carbohydrates obtainable from grape, and 5-15% (w/w) carbohydrates obtainable from pear, wherein the percentages are based on the dry matter.

In another preferred embodiment the composition comprises carbohydrates obtainable from carob, apple, grape, pear, peach, orange and lemon, in particular 30-40% (w/w) carbohydrates obtainable from carob, 35-45% (w/w) carbohydrates obtainable from apple, 5-15% (w/w) carbohydrates obtainable from grape, 5-15% (w/w) carbohydrates obtainable from pear, 1-3% (w/w) carbohydrates obtainable from peach, 1-3% (w/w) carbohydrates obtainable from orange, and 0.5-1.5% (w/w) carbohydrates obtainable from lemon, wherein the percentages are based on the dry matter.

In a particular preferred embodiment the composition comprises 35% (w/w) carbohydrates obtainable from carob, 40% (w/w) carbohydrates obtainable from apple, 10% (w/w) carbohydrates obtainable from white grape, 10% (w/w) carbohydrates obtainable from pear, 2% (w/w) carbohydrates obtainable from peach, 2% (w/w) carbohydrates obtainable from orange, and 1% (w/w) carbohydrates obtainable from lemon, based on the dry matter.

The term carbohydrate as used herein designates chemical compounds containing oxygen, hydrogen, and carbon atoms, in particular sugars such as mono- and di-saccharides, and polyalcohols.

The composition comprises glucose, fructose, saccharose, polyalcohols, and other sugars, in particular 18-28% (w/w) of glucose, 30-44% (w/w) of fructose, 16-33% (w/w) of saccharose, 7-13% (w/w) of polyalcohols, and 1-3% (w/w) of other sugars, wherein the percentages are based on the dry matter. These ranges depend on the specific fruits used for preparing the composition (e.g., variety, crop features) and are subject to biological variations.

In a preferred embodiment the composition of the invention is in the form of a syrup. If the composition is present in the form of a syrup the water content is preferably 21-31% (w/w).

The composition of the invention is prepared by a process comprising
a. obtaining a composition comprising carbohydrates from carob,
b. obtaining a composition comprising carbohydrates from at least one further fruit, and
c. and mixing the products of steps a) and b).

The composition obtained in step a) comprises preferably carbohydrates form carob pulp. The carob pulp used in the process is preferably cut, seedless and sieved to a homogeneous size. The size of the seedless carob pulp is preferably 10-20 mm. The moisture content of the seedless carob pulp is preferably 5 to 15 %.

The carob can be extracted as described in U.S. patent 5,624,500. The content of soluble solids expressed in °Brix ranges preferably from about 16-22°Brix in the obtained raw extract. The pH of the raw carob extract is preferably between 4.6 - 5.8.

Preferably the aqueous carob extract is decanted and centrifuged, and the insoluble parts are removed in step a) of the process. The carob extract is preferably first decanted and subsequently centrifuged. The centrifuge is preferably a vertical centrifuge. The content of initial insoluble solids of the raw carob extract is preferably reduced until less than 0.8 % (v/v).

In a further preferred embodiment, this aqueous carob extract is pasteurized and/or clarified by means of ultrafiltration. A plate-heat exchanger is preferably used for pasteurisation. The temperature for pasteurisation is preferably 85 +/-2°C. The pasteurisation time is preferably at least 20 seconds.

The clarification is preferably performed by ultrafiltration with a ceramic membrane which preferably has a porous size of about 75,000 Daltons (molecular weight cut-off). The temperature during clarification is preferably below 40°C. The clear carob extract has preferably 14-20°Brix.

In a preferred embodiment of the process the at least one fruit in step b) is selected from the group consisting of apple, white grape, pear, peach, orange, and lemon. Preferably, carbohydrates are obtained from orange pulp in step b).

The orange pulps are preferably milled, water extracted and pressed in step b) of the process. The milling of the orange pulps is preferably performed by a hammer-mill. The particle size of the orange pulp after milling is preferably 2-3 mm. The ratio of water to orange pulp for the water extraction is preferably around 2.5:1. The extraction lasts preferably about 30 minutes. The extraction is preferably a continuous extraction. For pressing a belt press is preferably used.

The obtained aqueous extract of orange has preferably between 3-5°Brix. The pulp content of the aqueous extract is preferably around 10-15% (v/v). The pH of the aqueous extract is preferably <4.0. This aqueous is preferably decanted and centrifuged, and the insoluble parts are removed. The initial pulp content of the raw orange extract is preferably until 1-2 % (v/v).

In a further preferred embodiment this extract is pasteurized and/or clarified by ultrafiltration. Pasteurisation is preferably performed in a plate-heat exchanger. The pasteurisation temperature is preferably 85 +/-2°C. The pasteurisation time is preferably at least 20 seconds. The clarification is preferably performed by ultrafiltration with a membrane having a porous size of about 75,000 Daltons (molecular weight cut-off). The membrane is preferably a ceramic membrane. The temperature during clarification is preferably below 40°C. The normal contents of soluble solids of the clear orange extract ranges preferably from 2-4°Brix.

The clear orange extract is preferably concentrated to 60-65°Brix. The concentration is preferably performed in a falling-film evaporator under vacuum conditions.

The composition in step b) of the process comprises preferably carbohydrates in the form of a fruit juice concentrate. The fruit juice concentrate is preferably a clear fruit juice concentrate and has the following analytical characteristics:
The clear apple juice concentrate has preferably 69 - 71°Brix.
The clear white grape juice concentrate has preferably 64 - 66°Brix.
The clear pear juice concentrate has preferably 69 - 71°Brix.
The clear peach juice concentrate has preferably 64 - 66°Brix.
The clear orange juice concentrate has preferably 64 - 66°Brix.
The clear lemon juice concentrate has preferably 47 - 50°Brix.

Such clear fruit juice concentrates are commercially available.

The mixture of step c) of the process has preferably a soluble solid concentration between 33-55°Brix. This mixture is preferably concentrated in a falling-film evaporator under vacuum conditions. For storage, the mixture is preferably concentrated to 69-71°Brix. The mixture is preferably stored at 0-5°C.

The mixture is preferably refined and decoloured by cation exchange chromatography, anion exchange chromatography, and/or adsorption chromatography. To this end, the mixture is preferably diluted with water to 20-25°Brix. A strongly acid cation exchange resin in the hydrogen form (activated) is preferably used for cation exchange chromatography. A weakly basic anion exchange resin in the hydroxide form (activated) is preferably used for anion exchange chromatography. A polymeric absorbent resin is preferably used for decolouring. In a preferred embodiment the mixture is subjected to cation exchange chromatography and anion exchange chromatography, more preferably to cation exchange chromatography, anion exchange chromatography and adsorption chromatography. Preferably, the mixture is first subjected to cation exchange chromatography, then to anion exchange chromatography, and subsequently to adsorption chromatography. The conductivity value of the mixture after adsorption chromatography is preferably at most 120 MicroSiemens/cm at 25°C.

The refined and decoloured mixture is preferably concentrated. The concentration is preferably performed in a falling-film evaporator under vacuum conditions. The mixture is preferably concentrated to 69-79°Brix.

The concentrated mixture is preferably subjected to pasteurisation. The pasteurisation is preferably performed in a plate-heat exchanger. The pasteurisation temperature is preferably 90.5 - 92.5°C. The pasteurisation time is preferably 45 seconds.

After pasteurisation, the concentrated mixture is preferably cooled to 10-25°C. In a preferred embodiment, the mixture is then filtered, in particular with filter having a mesh size of about 60 microns.

For storage, the concentrate is preferably cooled to 0-5°C.

The composition of the invention has preferably one or more, most preferably all of the following characteristics:
The °Brix at 20°C ranges preferably from 69 to 79°Brix.
The pH at 20°C (dilution at 10-16°Brix) is preferably 3.5-4.5.
The total acidity (expressed in g/kg of concentrate) is preferably at most 1.5 g/kg (in tartaric acid).
The colour (E420nm, dilution at 10°Brix, d=1 cm) is preferably at most 0.020.
The conductivity (MicroSiemens/cm at 25°C, in a dilution at 25°Brix) is preferably at most 150.
The Formol Index (expressed as ml NaOH 0.1N/100 ml dilution at 14°Brix) is preferably at most 1.5.
The turbidity (NTU in a dilution at 10°Brix) is preferably at most 1.5.
The composition of the invention is preferably sensorically odourless, colourless and just sweet (dilution at 10 - 16°Brix).

The composition of the invention is GMO-free, allergen-free, pesticide-free and heavy metals-free.

The term "osmotised water" as used herein designates water with a maximum conductivity of 10 MicroSiemens/cm at 25°C.

For the purposes of this invention, the term "extracting" is meant to comprise any separation method from a solid or liquid mixture whether it is conducted with or without a solvent.

For the purposes of this invention, the ultrafiltration, the ion exchange and the absorption (decolouring) chromatography in the process for the preparation of the composition can be performed as described in U.S. Patent 6,709,527.

The so-called refining is an important step in the preparation of the composition of the invention. The refining process enables the removal of essentially all the characteristic substances from the mentioned fruit sources except the sugar and the polyalcohol species (target).

The process for preparing the composition does not involve an enzymatic or chemical step for converting carbohydrates such as oligosaccharides or polysaccharides (e.g., starch, hemicellulose, pectins) to mono- or di-saccharides or the corresponding carbohydrate monomeric units.

The composition can be used for the preparation of foodstuff, in particular a beverage, confectionery, bakery product, dairy product, ice cream or chocolate.

The composition of the invention is preferably used for the manufacture of a soft-drink, a juice beverage, a nectar, a dairy product (e.g., a whey product or a yogurt, such as a fruit yogurt or a drink yogurt), a bakery product (e.g., a cookie, a muffin, a cereal bar, a fruit bar, or a cereal breakfast coating), a confectionery (e.g., a jelly, a soft candy, a pectin-jelly candy, or a hard-boiled candy), an ice cream, or chocolate (e.g., a chocolate bar or a chocolate sauce).

More specifically, the composition of the present invention can be used for the following foodstuff:
- Soft-Drinks
   Beverages (sparkling or still).
   Drinks, such as juice-containing drinks.
   Nectars, by replacing the used regular sugars.
   Clear or cloudy beverages for all the aforementioned categories.
- Tea Drinks
   By replacing the used regular sugars.
- Functional Drinks
   By replacing the used regular sugars and also being combined with artificial sweeteners (e.g., saccharin, acetosulfame, aspartame, cyclamate, or sucralose).
- Sport Drinks
   By replacing the used regular sugars.
- Herbal Drinks
   By replacing the used regular sugars and also being combined with artificial sweeteners (e.g., saccharin, acetosulfame, aspartame, cyclamate, or sucralose).
- CONFECTIONERY: by replacing the used regular sugars.
   - Jelly Gums.
   - Soft Candies.
   - Pectin Candies / Pectin Jellies.
   - Chewing Gums.
   - Hard Boiled Candies.
- CHOCOLATES: by replacing the used regular sugars.
   - Dark Chocolate bars.
   - Chocolate sauces.
   - Chocolate Pralinés: the chocolate base and the fillings or also the fruit fillings.
   - Fruit Preparations, e.g., for fillings, toppings etc. of the chocolate products; by replacing the used regular sugars.
- BAKERY: by replacing the used regular sugars.
   - Soy Bars.
   - Cereal Bars.
   - Fruit Bars: optionally with fillings such as fruit fillings
   - Muffins.
   - Breakfast Cereal Coatings.
   - Biscuits: optionally with fillings such as fruit fillings.
   - Cookies: optionally with toppings such as fruit toppings.
   - Fruit Preparations, e.g. for fillings, toppings, etc. of the bakery products; by replacing the used regular sugars.
- ICE CREAMS, WATER ICE & SORBETS: by replacing the used regular sugars. No fat, low fat and whole fat categories.
- DAIRY: by replacing the used regular sugars. No fat, low fat and whole fat categories.
   - White Yogurts.
   - Fruit Yogurts.
   - Drink Yogurts.
   - Fruit Drink Yogurts.
   - Whey Drink.
   - Fruit Whey Drink.
   - All the Fruit Preparations to be used in the aforementioned dairy products: by replacing the used regular sugars.
- JAMS / MARMELADES: by replacing the used regular sugars.

The composition of the invention represents a semi-finished product and allows for the preparation of low GI foodstuff. The composition of the invention significantly helps to reduce the GI of the foodstuff. However, since the GI of the final product may be altered by further ingredients, the final product will not necessarily have a low GI.

In a further embodiment the invention is directed to a pharmaceutical composition comprising the composition of the invention. The composition of the invention is preferably used for the prevention or treatment of type 2 diabetes, cardiovascular disease, coronary heart disease, obesity or hyperlipidemia.

The composition of the invention has been subjected to GI - clinical tests in several specialised centres (e.g., Oxford Brookes University, Profil Institute Neuss, Freiburg University) in order to determine its GI and the GI of some foodstuff applications. These tests indicated a GI ranging from 34 to 42, depending on the used fruit percentage of the formula. The tests were performed following an official method specified by the FAO/WHO ("Carbohydrates in Human Nutrition". FAO Food and Nutrition Paper 66. Report of a Joint FAO/WHO Expert Consultation. FAO, Rome 1998).

The clinical tests show that the composition of the invention is suitable for treating or preventing type 2 diabetes, cardiovascular disease, coronary heart disease, obesity or hyperlipidemia.

In beverage/drink applications the GI as determined by the clinical tests ranged from 34 to 54, depending on the recipe. These results were obtained for applications wherein the composition of the invention was used together with other food ingredients, even if other carbohydrate sources (e.g. fruit juice concentrates) were used.

In all cases the obtained GIs were classified as low. Thus, the composition of the invention significantly lowers the GI of foodstuff when it is applied in foodstuff, in comparison with the same foodstuff formulated and prepared by using regular sugar sources (e.g., glucose syrup, crystal sugar-sucrose).

The composition allows for the preparation of foodstuff with surprisingly low GI.

A beverage/drink application having a GI of 34 can be prepared for example from a composition comprising 40 % (w/w) carbohydrates obtainable from carob, 45 % (w/w) carbohydrates obtainable from apple, and 15 % (w/w) carbohydrates obtainable from grape, wherein the percentages are based on the dry matter.

A beverage/drink application having a GI of 42 can be prepared for example from a composition comprising 35 % (w/w) carbohydrates obtainable from carob, 40 % (w/w) carbohydrates obtainable from apple, 10 % (w/w) carbohydrates obtainable from grape, 10 % (w/w) carbohydrates obtainable from pear, 2 % (w/w) carbohydrates obtainable from peach, 2 % (w/w) carbohydrates obtainable from orange, and 1 % (w/w) carbohydrates obtainable from lemon, wherein the percentages are based on the dry matter.

The composition can replace completely or partially the used regular sugars in food applications (e.g., crystal sugar or saccharose, fructose syrup, glucose-fructose syrups, glucose syrups).

Drinks/beverages prepared from the composition of the invention are preferably diluted for human consumption until the proper dilution factor based on the desired sweetening strength and/or sensorial targets (e.g., with water, fruit juices, or other liquid ingredients).

In case of other food systems (e.g., confectionery, bakery, dairy, ice cream, chocolate), the composition of the invention can be applied directly (in concentrate form) or blended with other characteristic ingredients of the specific food application during the process.

In all the cases, the dosage depends on the required sweetening strength/profile or the targeted final taste.

The composition of the invention has a well-balanced sweet taste and a particular mouthfeel. Further, the taste is weakly fruity and full-bodied compared with regular sugars used in the food industry (e.g., crystal sugar, glucose syrup, invert sugar syrup, fructose syrup).

The composition of the invention is free of odour and colour.

The production process of the fruit extracts and the composition of the invention will be described in the following with reference to preferred embodiments.
- A. Description of preferred production process of fruit extracts of carob and orange
- B. Description of preferred production process of the composition of the invention from mixed fruit extracts

In the following the preferred steps of preparing the claimed composition are described in more detail.

### A.- FRUIT EXTRACTS

All the material and technological aids used are strictly "food grade" approved.

### A.1. CAROB EXTRACT

### A.1.1 Incoming Raw Material: Seedless Carob Pulp

Seedless carob pulp is preferably used as raw material for the carob extract production. The seedless carob pulp has preferably an average particle size of 10 - 20 mm and a moisture content of 5 - 15 %.

The average composition of the seedless carob pulp is as follows:

| | |
|---|---|
| Fats (%) | 0.14 |
| Proteins (%) | 3 - 4 |
| Soluble Carbohydrates (%) | 40 - 50 |
| Hemicelluloses (%) | 17 - 19 |
| Lignine (%) | 18 - 20 |
| Celluloses (%) | 8 - 10 |
| Ash (%) | 2 - 3 |
| Tannins (Polyphenols) (%) | 1 - 3 |
| Magnesium (ppm) | 225 - 250 |
| Calcium (ppm) | 2800 - 3000 |
| Potassium (ppm) | 3100 - 3400 |

The percentages are expressed in weight of the dry matter.

### A.1.2 Extraction

For the purposes of this invention, the carob can be extracted as described in U.S. Patent 5,624, 500.

The content of soluble solids expressed as °Brix ranges preferably from about 16 - 22°Brix in the obtained raw carob extract.

The regular pH of the raw carob extract is preferably between 4.6 - 5.8.

### A. 1.3 Decantation - Centrifugation

By using decanters (first) and vertical centrifuges (second), the content of initial insoluble solids of the raw carob extract is preferably reduced until less than 0.8 % (v/v).

### A. 1.4 Pasteurisation

Micro-stability and inactivation of endogenous enzymes are the main targets of this step.

It is preferably achieved in a regular plate-heat exchanger pasteurisation unit with the following thermal parameters:
- Temperature: 85 +/- 2°C
- Time: minimum 20 seconds.

Once the raw carob extract has been pasteurised, it is preferably rapidly chilled until 15 - 20°C.

### A.1.5 Clarification

The Clarification process of the raw carob extract is achieved by means of ultrafiltration (U-F).

The used U-F equipment has preferably a membrane porous size of about 75,000 Daltons (molecular weight cut-off), ceramic membranes, vertical configuration.

The used membranes are preferably "food grade approved" by conforming to 3-A Sanitary Standards for cross-flow membranes modules: 45-02 and under authorisation number 1181.

The maximum working temperature of the filtration system is preferably below 40°C on the product.

The U-F permits obtaining a clear-brilliant-transparent clear carob extract, with dark brownish colour and typical carob odour. The normal content of soluble solids of this clear liquid fraction ranges preferably from 14 - 20°Brix.

The permeate - clear carob extract (14 - 20°Brix) is preferably stored in tanks for being mixed with clear fruit juice concentrate and/or a clear orange extract concentrate (produced according to chapter A.2).

### A.2. ORANGE EXTRACT

### A.2.1 Incoming Raw Material: Orange pulp

Orange pulp is the used raw material for the orange extract production. The incoming orange pulp contains different non-edible parts of the orange fruit once the juice has been obtained by squeezing the orange: albedo, flavedo, juice sacs are the main constituents of this raw material.

The orange pulp is preferably initially checked in terms of moisture/humidity (average: 75 - 85% humidity) and sound aspect (e.g., free of non-orange material, no fermented pulp, typical fresh orange pulp odour).

### A.2.2 Milling - Grinding

The orange pulp is preferably transported through screw-conveyors for feeding two hammer-mill equipments which are crushing, grinding and sieving the orange pulp until a regular particle size of 2 - 3 mm is achieved.

### A.2.3 Extraction - Pressing

The milled-ground orange pulp (of the step A.2.2) is preferably fed into a tank and mixed with osmotised water at the ratio of "water: orange" of around 2.5 : 1. The tank contains preferably a stirrer which facilitates the homogeneous extraction of the orange pulp's soluble components. After about 30 minutes of continuous extraction and stirring, a homogenous and thick orange paste is obtained.

The extraction is preferably completed by feeding the orange paste to a belt-press which enables to separate a liquid fraction (named raw orange extract) and a solid fraction (named orange press-cake). Orange press-cake is considered as a waste material.

The raw orange extract is preferably a very pulpy/cloudy orange coloured liquid with between 3-5°Brix. The pulp content is preferably around 10-15 % (v/v). The pH is preferably < 4.0.

### A.2.4 Decantation - Centrifugation

By using proper decanters (first) and vertical centrifuges (second), the initial pulp content of the raw orange extract is preferably reduced until 1-2% (v/v).

### A.2.5 Pasteurisation

Pasteurisation is preferably achieved in a regular plate-heat exchanger pasteurisation unit with the following thermal parameters:
- Temperature: 85 +/- 2°C
- Time: minimum 20 seconds.

The main target of this step is to increase the microstability.

Once the orange extract has been pasteurised, it is preferably rapidly chilled until 15 - 20°C.

### A.2.6 Clarification

The clarification process of the "pulp reduced" orange extract is achieved by means of ultrafiltration (U-F).

For the purpose of this invention, this clarification step by using ultrafiltration can be performed as described in chapter A. 1.5.

The U-F permits obtaining a clear-brilliant-transparent clear orange extract, with yellowish colour and typical orange odour. The normal contents of soluble solids of this clear liquid fraction ranges preferably from 2 - 4°Brix.

The permeate, i.e., the clear orange extract, is preferably stored in tanks to be rapidly processed.

### A.2.7 Concentration and Storage

The clear orange extract is preferably concentrated in a falling-film evaporator under vacuum conditions.

The clear orange extract is preferably concentrated from 2 - 4°Brix until between 60 - 65°Brix.

Finally, the obtained clear orange extract concentrate is preferably stored in tanks in cool conditions (0 - 5°C) for a further processing step.

### A.3. BLENDING / MIXING THE CLEAR CAROB EXTRACT WITH CLEAR ORANGE EXTRACT CONCENTRATE AND/OR FRUIT JUICE CONCENTRATES AND CONCENTRATION: Clear Fruit Concentrate Blend.

### A.3.1 Blending / Mixing

The clear carob extract with preferably between 14 - 20°Brix (see chapter A.1) is mixed with:
- clear orange extract concentrate with preferably between 60 - 65°Brix (see chapter A.2), and/or
- with at least one clear fruit juice concentrate.

The clear fruit juice concentrates have preferably the following analytical characteristics:

| **Clear Fruit Juice Concentrate** | **°Brix at 20°C** |
|---|---|
| Clear Apple Juice Concentrate | 69 - 71 |
| Clear White Grape Juice Concentrate | 64 - 66 |
| Clear Pear Juice Concentrate | 69 - 71 |
| Clear Peach Juice Concentrate | 64 - 66 |
| Clear Orange Juice Concentrate | 64 - 66 |
| Clear Lemon Juice Concentrate | 47 - 50 |

These clear fruit juice concentrates are commercially available.

After blending or mixing, the product reaches preferably a soluble solid concentration between 33 - 55°Brix.

### A.3.2 Concentration and Cool Storage

The product blend/mix obtained in the foregoing step and belonging at the chapter A.3.1 is now preferably concentrated in a falling-film evaporator under vacuum conditions.

The product blend/mix is preferably concentrated from 33 - 55°Brix until between 69 - 71°Brix.

Finally, the obtained product blend/mix is preferably stored in tanks in cool conditions (0 - 5°C) for a further processing step.

### B.- THE COMPOSITION

B.1 Dilution with osmotised water.

The product blend/mix produced in the foregoing chapter A.3.2 is preferably diluted with osmotised water until between 20 - 25°Brix.

B.2 Refining and decolouring: ion-exchange and adsorption technology (respectively).

Refining and decolouring is preferably performed as follows:
- First: Cation exchange treatment by using activated cationic resins.
- Second: Anion exchange treatment by using activated anion resins.
- Third: Adsorption/decolouring treatment by using activated polymeric resins.

Preferably, a continuous conductivity monitoring "in line" is providing conductivity data (expressed in MicroSiemens/cm at 25°C).

The conductivity value is preferably at most 120 MicroSiemens/cm at 25°C, measured in the outlet of the adsorption/decolouring resin column (third step of the treatment).

The de-ionised and decoloured product is preferably stored in tanks.

### B.3 Concentration and Storage

The de-ionised and decoloured product obtained in the foregoing step (see chapter B.2) is preferably concentrated in a falling-film evaporator under vacuum conditions.

The de-ionised and decoloured product is preferably concentrated from 15 - 20°Brix until between 69 - 79°Brix.

Finally, the concentrated product is preferably stored in tanks in cool conditions (0 - 5°C) for the further final processing steps, preferably pasteurization, filtration and final storage.

### B.4 Pasteurisation, Filtration, Final Storage

Before the filling-up or packaging, the composition is preferably microbiologically stabilised by:
- Pasteurisation: 90.5 - 92.5°C during minimum 45 seconds.
- Cooling/Chilling: 10 - 25°C.
- Filtration: 60 microns (mesh screen size).

The pasteurisation equipment consists preferably in a plate-heat exchanger and a holding system.

The cooling process is preferably performed with a plate-heat exchanger.

The composition of the invention is preferably stored in cool conditions (cold warehouse) at 0 - 5°C.

The composition of the invention is sensorically odourless and nearly colourless (from colourless until a very weak pale yellow colour in concentrated form, but colourless in diluted form, e.g., 10 -16°Brix).

The composition of the invention has preferably one or more, most preferably all of the following physico-chemical characteristics:
- The °Brix at 20°C ranges preferably from 69 to 79°Brix.
- The pH at 20°C (dilution at 10 -16°Brix) is preferably 3.5 - 4.5.
- The total acidity (expressed in g/kg of concentrate) is preferably at most 1.5 g/kg (in tartaric acid).
- The colour (E420nm, dilution at 10°Brix, d=1 cm) is preferably at most 0.020.
- The conductivity (MicroSiemens/cm at 25°C, in a dilution at 25°Brix) is preferably at most 150.
- The Formol Index (ml NaOH 0,1 N/100 ml dilution at 14°Brix) is preferably at most 1.5.
- The turbidity (NTU in a dilution at 10°Brix) is preferably at most 1.5.

Due to the process, the raw material origin and traceability, this product category is GMO-free and allergen-free. Also, due to the process (ion-exchange and adsorption technology), this product is pesticide-free and heavy metals-free.

The composition of the invention fulfills the microbiological and pathogen-free standards of the Fruit Juice Concentrates and Sugar Syrups industries.

### Examples

The weight percentages in the following examples are based on the dry matter. The final products of the following examples show a low GI.

### Example 1

### Composition of the invention

40 % (w/w) carbohydrates obtainable from carob
45 % (w/w) carbohydrates obtainable from apple
15 % (w/w) carbohydrates obtainable from grape

### Example 2

### Composition of the invention

35 % (w/w) carbohydrates obtainable from carob
40 % (w/w) carbohydrates obtainable from apple
10 % (w/w) carbohydrates obtainable from grape
10 % (w/w) carbohydrates obtainable from pear
2 % (w/w) carbohydrates obtainable from peach
2 % (w/w) carbohydrates obtainable from orange
1 % (w/w) carbohydrates obtainable from lemon

### Example 3

### **Soft drinks just sweetened with composition of the invention

1 part of composition + 5 parts of water.................. regular sweetening strength
1 part of composition + 3.5 parts of water................high sweet taste.

### Example 4

### **Orange nectar (50 % juice content) sweetened with composition of the invention instead of crystal sugar (sucrose or saccharose)

- At industrial scale: to obtain 1000 I
   90 kg of orange juice concentrate having 65°Brix.
   83.5 kg of composition having 70°Brix.
   871.5 kg of water.
- Made at home:
   500 ml or 522,5 g of orange juice having 11.2°Brix.
   80 g of composition having 70°Brix.
   422.5 g of water or enough water until 1 I final volume.

### Example 5

### **White Yogurt sweetened with composition of the invention

- Whole fat (3.5 %) White Yogurt:
   9 - 10 g of composition having 78°Brix
   90 - 91 g of white yogurt base
- Low fat (1.5 %) White Yogurt:
   15 -16 g of composition having 78°Brix
   84 - 85 g of white yogurt base

## Claims

1. A composition comprising carbohydrates obtainable from carob and carbohydrates obtainable from at least one further fruit, comprising 18-28% (w/w) of glucose, 30-44% (w/w) of fructose, 16-33% (w/w) of saccharose, 7-13% (w/w) of polyalcohols, and 1-3% (w/w) of other sugars, wherein the percentages are based on the dry matter.

2. The composition of claim 1, comprising carbohydrates obtainable from carob and apple.

3. The composition of claim 2, comprising 40-60% (w/w) carbohydrates obtainable from carob, and 40-60% (w/w) carbohydrates obtainable from apple, wherein the percentages are based on the dry matter.

4. The composition of any of claims 1 to 3, comprising carbohydrates obtainable from carob, apple and grape.

5. The composition of claim 4, comprising 35-45% (w/w) carbohydrates obtainable from carob, 40-50% (w/w) carbohydrates obtainable from apple, and 10-20% (w/w) carbohydrates obtainable from grape, wherein the percentages are based on the dry matter.

6. The composition of any of claims 1 to 5, comprising carbohydrates obtainable from carob, apple, grape and pear.

7. The composition of claim 6, comprising 35-45% (w/w) carbohydrates obtainable from carob, 35-45% (w/w) carbohydrates obtainable from apple, 5-15 % (w/w) carbohydrates obtainable from grape, and 5-15% (w/w) carbohydrates obtainable from pear, wherein the percentages are based on the dry matter.

8. The composition of any of claims 1 to 7, comprising carbohydrates obtainable from carob, apple, grape, pear, peach, orange and lemon.

9. The composition of claim 8, comprising 30-40% (w/w) carbohydrates obtainable from carob, 35-45% (w/w) carbohydrates obtainable from apple, 5-15% (w/w) carbohydrates obtainable from grape, 5-15% (w/w) carbohydrates obtainable from pear, 1-3% (w/w) carbohydrates obtainable from peach, 1-3% (w/w) carbohydrates obtainable from orange, and 0.5-1.5% (w/w) carbohydrates obtainable from lemon, wherein the percentages are based on the dry matter.

10. A process for the preparation of the composition according to any of claims 1 to 9, comprising
a. obtaining a composition comprising carbohydrates from carob,
b. obtaining a composition comprising carbohydrates from at least one further fruit, and
c. and mixing the products of steps a) and b).

11. The process of claim 10, wherein in step a) the composition comprises carbohydrates from carob pulp.

12. The process of claim 11, wherein in step a) the carob pulp is extracted with water.

13. The process of claim 12, wherein in step a) the aqueous extract of carob pulp is decanted and centrifuged, and the insoluble parts are removed.

14. The process of claim 12 or 13, wherein in step a) the aqueous extract of carob pulp is pasteurised.

15. The process of any of claims 12 to 14, wherein in step a) the aqueous extract of carob is clarified by means of ultrafiltration.

16. The process of any of claims 12 to 15, wherein in step a) the aqueous extract of carob has 14 - 20'Brix.

17. The process of any of claims 10 to 16, wherein in step b) the at least one fruit is selected from the group consisting of apple, white grape, pear, peach, orange and lemon.

18. The process of claim 17, wherein in step b) carbohydrates are obtained from orange pulp.

19. The process of claim 18, wherein in step b) the orange pulp is extracted with water.

20. The process of claim 18 or 19, wherein in step b) the orange pulp is milled, extracted with water and pressed, and the insoluble parts are removed.

21. The process of claim 19 or 20, wherein in step b) the aqueous extract of orange pulp is decanted and centrifuged, and the insoluble parts are removed.

22. The process of any of claims 19 to 21, wherein in step b) the aqueous extract of orange pulp is pasteurised.

23. The process of any of claims 19 to 22, wherein in step b) the aqueous extract of orange pulp is clarified by ultrafiltration.

24. The process of any of claims 19 to 23, wherein in step b) the aqueous extract of orange pulp is concentrated until 60-65°Brix.

25. The process of any of claims 10 to 24, wherein the composition in step b) comprises carbohydrates in the form of a fruit juice concentrate.

26. The process of any of claims 10 to 25, wherein the mixture in step c) is refined and decoloured by cation exchange chromatography, anion exchange chromatography, and/or adsorption chromatography.

27. The process of any of claims 10 to 26, wherein the mixture in step c) is concentrated until 69-79°Brix.

28. Use of the composition of any claims 1 to 9 for the preparation of foodstuff.

29. The use according to claim 28, wherein the foodstuff is a beverage, confectionery, bakery product, dairy product, ice cream or chocolate.

30. Pharmaceutical composition comprising the composition of any of claims 1 to 9.

31. Composition of any of claims 1 to 9 for use in the prevention or treatment of type 2 diabetes, cardiovascular disease, coronary heart disease, obesity or hyperlipidemia.

32. Use of the composition of any of claims 1 to 9 for the preparation of a medicament for the prevention or treatment of type 2 diabetes, cardiovascular disease, coronary heart disease, obesity or hyperlipidemia.

## Patentansprüche

1. Zusammensetzung umfassend Kohlenhydrate, die aus Johannisbrot erhältlich sind, und Kohlenhydrate, die aus wenigstens einer weiteren Frucht erhältlich sind, umfassend 18-28 % (Gew./Gew.) Glucose, 30-44 % (Gew./Gew.) Fructose, 16-33 % (Gew./Gew.) Saccharose, 7-13% (Gew./Gew.) Polyalkohole, und 1-3% (Gew./Gew.) andere Zucker, wobei sich die Prozentsätze auf die Trockensubstanz beziehen.

2. Zusammensetzung nach Anspruch 1, umfassend Kohlenhydrate, die aus Johannisbrot und Apfel erhältlich sind.

3. Zusammensetzung nach Anspruch 2, umfassend 40-60 % (Gew./Gew.) Kohlenhydrate, die aus Johannisbrot erhältlich sind, und 40-60 % (Gew./Gew.) Kohlenhydrate, die aus Apfel erhältlich sind, wobei sich die Prozentsätze auf die Trockensubstanz beziehen.

4. Zusammensetzung nach einem der Ansprüche 1 bis 3, umfassend Kohlenhydrate, die aus Johannisbrot, Apfel und Weintraube erhältlich sind.

5. Zusammensetzung nach Anspruch 4, umfassend 35-45 % (Gew./Gew.) Kohlenhydrate, die aus Johannisbrot erhältlich sind, 40-50 % (Gew./Gew.) Kohlenhydrate, die aus Apfel erhältlich sind, und 10-20 % (Gew./Gew.) Kohlenhydrate, die aus Weintraube erhältlich sind, wobei sich die Prozentsätze auf die Trockensubstanz beziehen.

6. Zusammensetzung nach einem der Ansprüche 1 bis 5, umfassend Kohlenhydrate, die aus Johannisbrot, Apfel, Weintraube und Birne erhältlich sind.

7. Zusammensetzung nach Anspruch 6, umfassend 35-45 % (Gew./Gew.) Kohlenhydrate, die aus Johannisbrot erhältlich sind, 35-45 % (Gew./Gew.) Kohlenhydrate, die aus Apfel erhältlich sind, 5-15% (Gew./Gew.) Kohlenhydrate, die aus Weintraube erhältlich sind, und 5-15 % (Gew./Gew.) Kohlenhydrate, die aus Birne erhältlich sind, wobei sich die Prozentsätze auf die Trockensubstanz beziehen.

8. Zusammensetzung nach einem der Ansprüche 1 bis 7, umfassend Kohlenhydrate, die aus Johannisbrot, Apfel, Weintraube, Birne, Pfirsich, Orange und Zitrone erhältlich sind.

9. Zusammensetzung nach Anspruch 8, umfassend 30-40 % (Gew./Gew.) Kohlenhydrate, die aus Johannisbrot erhältlich sind, 35-45 % (Gew./Gew.) Kohlenhydrate, die aus Apfel erhältlich sind, 5-15% (Gew./Gew.) Kohlenhydrate, die aus Weintraube erhältlich sind, 5-15 % (Gew./Gew.) Kohlenhydrate, die aus Birne erhältlich sind, 1-3 % (Gew./Gew.) Kohlenhydrate, die aus Pfirsich erhältlich sind, 1-3% (Gew./Gew.) Kohlenhydrate, die aus Orange erhältlich sind, und 0,5-1,5 % (Gew./Gew.) Kohlenhydrate, die aus Zitrone erhältlich sind, wobei sich die Prozentsätze auf die Trockensubstanz beziehen.

10. Verfahren zur Herstellung der Zusammensetzung gemäß einem der Ansprüche 1 bis 9, umfassend:
a) das Erhalten einer Zusammensetzung umfassend Kohlenhydrate aus Johannisbrot,
b) das Erhalten einer Zusammensetzung umfassend Kohlenhydrate aus wenigstens einer weiteren Frucht, und
c) das Vermischen der Produkte der Schritte a) und b).

11. Verfahren nach Anspruch 10, wobei in Schritt a) die Zusammensetzung Kohlenhydrate aus Johannisbrotpulpe umfasst.

12. Verfahren nach Anspruch 11, wobei in Schritt a) die Johannisbrotpulpe mit Wasser extrahiert wird.

13. Verfahren nach Anspruch 12, wobei in Schritt a) der wässrige Extrakt von Johannisbrotpulpe dekantiert und zentrifugiert wird und die unlöslichen Teile entfernt werden.

14. Verfahren nach Anspruch 12 oder 13, wobei in Schritt a) der wässrige Extrakt von Johannisbrotpulpe pasteurisiert wird.

15. Verfahren nach einem der Ansprüche 12 bis 14, wobei in Schritt a) der wässrige Extrakt von Johannisbrot mittels Ultrafiltration geklärt wird.

16. Verfahren nach einem der Ansprüche 12 bis 15, wobei in Schritt a) der wässrige Extrakt von Johannisbrot 14-20° Brix aufweist.

17. Verfahren nach einem der Ansprüche 10 bis 16, wobei in Schritt b) die wenigstens eine Frucht ausgewählt ist aus der Gruppe bestehend aus Apfel, weißer Traube, Birne, Pfirsich, Orange und Zitrone.

18. Verfahren nach Anspruch 17, wobei in Schritt b) Kohlenhydrate aus Orangenpulpe erhalten werden.

19. Verfahren nach Anspruch 18, wobei in Schritt b) die Orangenpulpe mit Wasser extrahiert wird.

20. Verfahren nach Anspruch 18 oder 19, wobei in Schritt b) die Orangenpulpe gemahlen, mit Wasser extrahiert und gepresst wird und die unlöslichen Teile entfernt werden.

21. Verfahren nach Anspruch 19 oder 20, wobei in Schritt b) der wässrige Extrakt von Orangenpulpe dekantiert und zentrifugiert wird und die unlöslichen Teile entfernt werden.

22. Verfahren nach einem der Ansprüche 19 bis 21, wobei in Schritt b) der wässrige Extrakt von Orangenpulpe pasteurisiert wird.

23. Verfahren nach einem der Ansprüche 19 bis 22, wobei in Schritt b) der wässrige Extrakt von Orangenpulpe durch Ultrafiltration geklärt wird.

24. Verfahren nach einem der Ansprüche 19 bis 23, wobei in Schritt b) der wässrige Extrakt von Orangenpulpe bis 60-65° Brix konzentriert wird.

25. Verfahren nach einem der Ansprüche 10 bis 24, wobei die Zusammensetzung in Schritt b) Kohlenhydrate in Form eines Fruchtsaftkonzentrats umfasst.

26. Verfahren nach einem der Ansprüche 10 bis 25, wobei die Mischung in Schritt c) durch Kationenaustauschchromatografie, Anionenaustauschchromatografie und/oder Adsorptionschromatografie raffiniert und entfärbt wird.

27. Verfahren nach einem der Ansprüche 10 bis 26, wobei die Mischung in Schritt c) bis 69-79° Brix konzentriert wird.

28. Verwendung der Zusammensetzung nach einem der Ansprüche 1 bis 9 für die Herstellung von Lebensmitteln.

29. Verwendung gemäß Anspruch 28, wobei das Lebensmittel ein Getränk, eine Süßware, eine Backware, ein Milchprodukt, eine Eiscreme oder Schokolade ist.

30. Pharmazeutische Zusammensetzung umfassend die Zusammensetzung nach einem der Ansprüche 1 bis 9.

31. Zusammensetzung nach einem der Ansprüche 1 bis 9 zur Verwendung bei der Verhütung oder Behandlung von Typ-2-Diabetes, Herz-Kreislauf-Erkrankung, koronarer Herzerkrankung, Fettleibigkeit oder Hyperlipidämie.

32. Verwendung der Zusammensetzung nach einem der Ansprüche 1 bis 9 für die Herstellung eines Medikaments für die Verhütung oder Behandlung von Typ 2-Diabetes, Herz-Kreislauf-Erkrankung, koronarer Herzerkrankung, Fettleibigkeit oder Hyperlipidämie.

## Revendications

1. Composition comprenant des glucides pouvant être obtenus à partir de caroube et des glucides pouvant être obtenus à partir d'au moins un fruit supplémentaire, comprenant 18-28% (p/p) de glucose, 30-44% (p/p) de fructose, 16-33 % (p/p) de saccharose, 7-13 % (p/p) de polyols, et 1-3 % (p/p) d'autres sucres, où les pourcentages sont basés sur la matière sèche.

2. Composition selon la revendication 1, comprenant des glucides pouvant être obtenus à partir de caroube et de pomme.

3. Composition selon la revendication 2, comprenant 40-60 % (p/p) de glucides pouvant être obtenus à partir de caroube, et 40-60 % (p/p) de glucides pouvant être obtenus à partir de pomme, où les pourcentages sont basés sur la matière sèche.

4. Composition selon n'importe lesquelles des revendications 1 à 3, comprenant des glucides pouvant être obtenus à partir de caroube, de pomme et de raisin.

5. Composition selon la revendication 4, comprenant 35-45 % (p/p) de glucides pouvant être obtenus à partir de caroube, 40-50 % (p/p) de glucides pouvant être obtenus à partir de pomme, et 10-20 % (p/p) de glucides pouvant être obtenus à partir de raisin, où les pourcentages sont basés sur la matière sèche.

6. Composition selon n'importe lesquelles des revendications 1 à 5, comprenant des glucides pouvant être obtenus à partir de caroube, de pomme, de raisin et de poire.

7. Composition selon la revendication 6, comprenant 35-45 % (p/p) de glucides pouvant être obtenus à partir de caroube, 35-45 % (p/p) de glucides pouvant être obtenus à partir de pomme, 5-15 % (p/p) de glucides pouvant être obtenus à partir de raisin, et 5-15 % (p/p) de glucides pouvant être obtenus à partir de poire, où les pourcentages sont basés sur la matière sèche.

8. Composition selon n'importe lesquelles des revendications 1 à 7, comprenant des glucides pouvant être obtenus à partir de caroube, de pomme, de raisin, de poire, de pêche, d'orange et de citron.

9. Composition selon la revendication 8, comprenant 30-40% (p/p) de glucides pouvant être obtenus à partir de caroube, 35-45 % (p/p) de glucides pouvant être obtenus à partir de pomme, 5-15 % (p/p) de glucides pouvant être obtenus à partir de raisin, 5-15 % (p/p) de glucides pouvant être obtenus à partir de poire, 1-3 % (p/p) de glucides pouvant être obtenus à partir de pêche, 1-3% (p/p) de glucides pouvant être obtenus à partir d'orange, et 0,5-1,5 % (p/p) de glucides pouvant être obtenus à partir de citron, où les pourcentages sont basés sur la matière sèche.

10. Procédé de préparation de la composition selon n'importe lesquelles des revendications 1 à 9, comprenant
a. l'obtention d'une composition comprenant des glucides provenant de caroube,
b. l'obtention d'une composition comprenant des glucides provenant d'au moins un fruit supplémentaire, et
c. et le mélange des produits des étapes a) et b).

11. Procédé selon la revendication 10, dans lequel à l'étape a) la composition comprend des glucides provenant de pulpe de caroube.

12. Procédé selon la revendication 11, dans lequel à l'étape a) la pulpe de caroube est extraite à l'eau.

13. Procédé selon la revendication 12, dans lequel à l'étape a) l'extrait aqueux de pulpe de caroube est décanté et centrifugé, et les parties insolubles sont éliminées.

14. Procédé selon la revendication 12 ou 13, dans lequel à l'étape a) l'extrait aqueux de pulpe de caroube est pasteurisé.

15. Procédé selon n'importe lesquelles des revendications 12 à 14, dans lequel à l'étape a) l'extrait aqueux de caroube est clarifié au moyen d'une ultrafiltration.

16. Procédé selon n'importe lesquelles des revendications 12 à 15, dans lequel à l'étape a) l'extrait aqueux de caroube est à 14-20°Brix.

17. Procédé selon n'importe lesquelles des revendications 10 à 16, dans lequel à l'étape b) ledit au moins un fruit est choisi dans le groupe constitué de la pomme, du raisin blanc, de la poire, de la pêche, de l'orange et du citron.

18. Procédé selon la revendication 17, dans lequel à l'étape b) les glucides sont obtenus à partir de pulpe d'orange.

19. Procédé selon la revendication 18, dans lequel à l'étape a) la pulpe d'orange est extraite à l'eau.

20. Procédé selon la revendication 18 ou 19, dans lequel à l'étape b) la pulpe d'orange est broyée, extraite à l'eau et pressée, et les parties insolubles sont éliminées.

21. Procédé selon la revendication 19 ou 20, dans lequel à l'étape b) l'extrait aqueux de pulpe d'orange est décanté et centrifugé, et les parties insolubles sont éliminées.

22. Procédé selon n'importe lesquelles des revendications 19 à 21, dans lequel à l'étape b) l'extrait aqueux de pulpe d'orange est pasteurisé.

23. Procédé selon n'importe lesquelles des revendications 19 à 22, dans lequel à l'étape b) l'extrait aqueux de pulpe d'orange est clarifié par ultrafiltration.

24. Procédé selon n'importe lesquelles des revendications 19 à 23, dans lequel à l'étape b) l'extrait aqueux de pulpe d'orange est concentré jusqu'à 60-65°Brix.

25. Procédé selon n'importe lesquelles des revendications 10 à 24, dans lequel la composition à l'étape b) comprend des glucides sous la forme d'un concentré de jus de fruits.

26. Procédé selon n'importe lesquelles des revendications 10 à 25, dans lequel le mélange à l'étape c) est raffiné et décoloré par une chromatographie d'échange de cations, une chromatographie d'échange d'anions, et/ou une chromatographie d'adsorption.

27. Procédé selon n'importe lesquelles des revendications 10 à 26, dans lequel le mélange à l'étape c) est concentré jusqu'à 69-79°Brix.

28. Utilisation de la composition selon n'importe lesquelles des revendications 1 à 9 pour la préparation d'un produit alimentaire.

29. Utilisation selon la revendication 28, où le produit alimentaire est une boisson, une confiserie, un produit de boulangerie, un produit laitier, une crème glacée ou du chocolat.

30. Composition pharmaceutique comprenant la composition selon n'importe lesquelles des revendications 1 à 9.

31. Composition selon n'importe lesquelles des revendications 1 à 9 pour une utilisation dans la prévention ou le traitement du diabète de type 2, des maladies cardiovasculaires, des coronaropathies, de l'obésité ou de l'hyperlipidémie.

32. Utilisation de la composition selon n'importe lesquelles des revendications 1 à 9 pour la préparation d'un médicament destiné à la prévention ou au traitement du diabète de type 2, des maladies cardiovasculaires, des coronaropathies, de l'obésité ou de l'hyperlipidémie.
